# EUROPEAN PATENT SPECIFICATION

(11) **EP 0 370 115 B1**
(45) Date of publication and mention of the grant of the patent: **14.09.1994**
(21) Application number: 89906472.9
(22) Date of filing: 06.06.1989
(51) Int. Cl.: A61B 1/00

(54) **CATHETER FOR DIAGNOSIS AND THERAPY**
KATHETER FÜR DIAGNOSE UND THERAPIE
CATHETER DE DIAGNOSTIC ET DE THERAPIE

(30) Priority: 06.06.1988 JP 138755/88
(43) Date of publication of application: 30.05.1990
(73) Proprietor: SUMITOMO ELECTRIC INDUSTRIES, LTD., Osaka-shi, Osaka 541 (JP)
(72) Inventor: KANAZAWA, Shin-ichi Osaka Works of Sumitomo Electr, Konohana-ku Osaka-shi Osaka 554 (JP); YOTSUYA, Koro Osaka Works of Sumitomo Electr., Konohana-ku Osaka-shi Osaka 554 (JP); SOGAWA, Ichiro Osaka Works of Sumitomo Electr., Konohana-ku Osaka-shi Osaka 554 (JP); UEMIYA, Takafumi Osaka Works of Sumitomo Electr., Konohana-ku Osaka-shi Osaka 554 (JP); NIWA, Shin-ichiro Osaka Works of Sumitomo Electr., Konohana-ku Osaka-shi Osaka 554 (JP)
(74) Representative: Füchsle, Klaus, Dipl.-Ing.
(86) International application number: JP8900570
(87) International publication number: WO8911818

(56) References cited:
- EP-A- 0 263 645
- DE-C- 3 447 642
- GB-A- 1 284 537
- JP-A- 5 172 194
- JP-A- 6 395 064
- US-A- 4 768 858

## Description

### TECHNICAL FIELD

The present invention generally relates to diagnostic and therapeutic catheters and more particularly, to a diagnostic and therapeutic catheter which is useful for performing endoscopy of vasa of living organisms, for example, blood vessels, oviducts, ureter, etc. or removing diseased parts in vasa by using laser beams.

### BACKGROUND ART

A diagnostic and therapeutic catheter is known into which optical fibers and tubes necessary for performing diagnosis and therapy are inserted alternately.

For example, in order to perform diagnosis and therapy of a diseased part in a blood vessel, an optical fiber for endoscopy and an optical fiber for illumination are initially inserted into the catheter and a balloon for securing a distal end portion of the catheter and for stopping blood flow, which is provided at the distal end portion of the catheter, is inflated so as to stop blood flow in the blood vessel. Then, liquid (flushing liquid) whose optical loss is small in a range of wavelength of laser beams in use is fed into the blood vessel so as to replace blood in the blood vessel such that a clear view in the blood vessel is obtained. At this time, a state in the blood vessel is displayed on a screen. Subsequently, when the diseased part in the blood vessel is found, endoscopy is stopped and the optical fiber for endoscopy is replaced by an optical fiber for irradiating laser beams. Thus, in this state, laser beams are irradiated onto the diseased part by the optical fiber for irradiating laser beams so as to remove the diseased part.

At this time, since stoppage of blood flow should be limited to a short time period in view of safety of the living organism, the above replacement of the optical fibers should be performed rapidly after endoscopy. Hence, if a long time is required for making the replacement of the optical fibers, stoppage of blood flow by the balloon should be cancelled and the operations which have been performed until then are required to be performed again.

Therefore, it may be appropriate to employ a catheter of a type in which a plurality of optical fibers and tubes are preliminarily accommodated up to a distal end portion of the catheter and diagnosis and therapy of a diseased part are performed by inserting the distal end portion of the catheter to vicinity of the diseased part. However, this catheter becomes large in diameter. Thus, this catheter has such a drawback that the catheter can be inserted into relatively thick vasa but cannot be inserted into thin vasa.

An object of the present invention is to provide, in view of the above described inconveniences, a diagnostic and therapeutic catheter by which diagnosis and therapy based on the above described operational procedures can be rapidly and positively performed even in thin vasa.

Earlier European application EP-A-0 338 149, that is considered to form part of the prior art under Article 54(3) and (4) EPC only, goes part way to solving the object of the present invention. It discloses fiberoptic systems and endoscopic devices each comprising a single fiber light guide which ia ring-shaped in cross-section. The hollow center region of this fiber light guide is used for receiving viewing bundles, laser transmitting fiber or fibers; transport channels, or other suitable devices therethrough. The transport channels can be used to deliver various solutions and medications into a body while performing a diagnostic function such as recanalization.

### DISCLOSURE OF THE INVENTION

The invention is as disclosed in the accompanying claims and in its widest sense comprises a diagnostic and therapeutic catheter accommodating a plurality of optical fibers and at least one tube in its proximal end. The distal end extent of the catheter is of reduced diameter compared with the diameter of the proximal end of the catheter. In the distal end extent of the catheter there is at least one duct. Said plurality of optical fibers and said at least one tube in said proximal end confront said duct in the distal end extent whereby any one of said optical fibers and said at least one tube is selectively retractably insertable into said at least one duct in said distal end extent.

In the catheter of the above described arrangement, a plurality of the optical fibers and tubes, which become required to be used sequentially, can be preliminarily set in a waiting state at a location relatively adjacent to a diseased part in a thin vas. Therefore, in case of necessity, a necessary one of the optical fibers and tubes can be selectively drawn close to the diseased part immediately by using the duct.

### BRIEF DESCRIPTION OF THE DRAWINGS

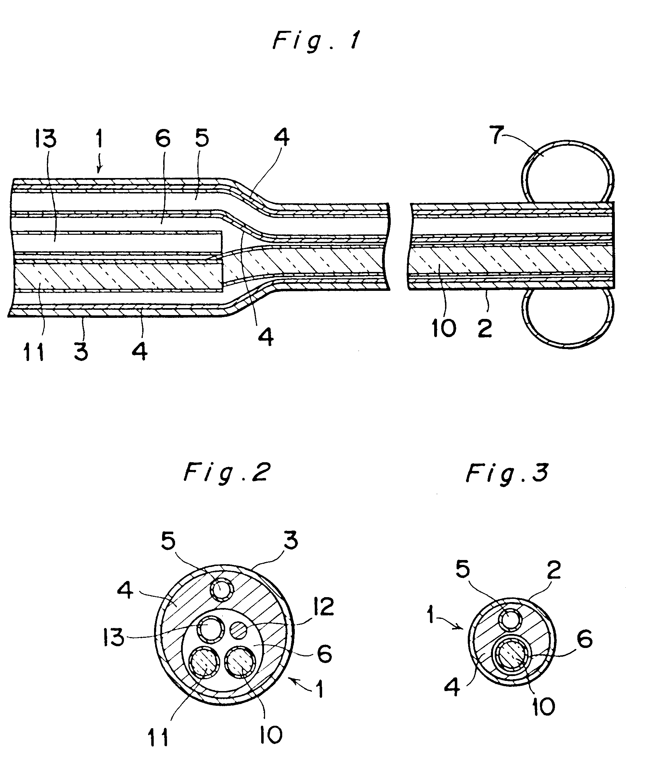

Fig. 1 is a longitudinal sectional view of a diagnostic and therapeutic catheter of the present invention, Fig. 2 is a transverse sectional view of a large diameter portion of the diagnostic and therapeutic catheter, and Fig. 3 is a transverse sectional view of a small diameter portion of the diagnostic and therapeutic catheter.

### BEST MODE FOR CARRYING OUT THE INVENTION

Hereinbelow, one embodiment according to the present invention will be explained by referring to the drawings. Fig. 1 is a sectional view of a diagnostic and therapeutic catheter 1 (referred to as a "catheter", hereinbelow) according to the present invention.

In Fig. 1, the catheter 1 includes a small diameter portion 2 of 10 to 20 cm in length. In the catheter 1, there are provided two ducts 5 and 6 extending continuously from a distal end of the small diameter portion 2 rearwards to a large diameter portion 3. A light guide 4 for illumination is formed in the catheter 1 so as to occupy a gap between peripheral surfaces of the ducts 5 and 6 (see Figs. 2 and 3).

The duct 5 is formed smaller in diameter than the duct 6 and is passed through the small diameter portion 2 at an identical diameter. The duct 5 is used as a gas passage for supplying gas to a balloon 7 fixed at the distal end of the small diameter portion 2.

The duct 6 is formed larger in diameter than the duct 5. The duct 6 is so formed in the small diameter portion 2 as to easily receive any one of an optical fiber 10 for transmitting laser beams, an image optical fiber 11 for endoscopy, a wire 12 for controlling the distal end portion, a passage 13 for discharging blood and supplying flushing liquid in place of blood, etc., which are accommodated in the catheter (see Fig. 3). On the other hand, the duct 6 is so formed in the large diameter portion 3 as to simultaneously receive the optical fiber 10, the image optical fiber 11, the wire 12, the passage 13, etc.

At a location which confronts an inlet of the small diameter portion 2 forwards from the duct 6, the optical fiber 10, the image optical fiber 11, the wire 12, the passage 13, etc. are retractably inserted into the duct in the small diameter portion 2 selectively. In this embodiment, the optical fiber 10 for transmitting laser beams is inserted from the inlet of the small diameter portion 2 to the distal end of the small diameter portion 2 so as to be used for performing laser therapy from the distal end adjacent to a diseased part as shown in Figs. 1 and 3 (see Fig. 3).

In the diagnostic and therapeutic catheter of the above described arrangement, as shown in Fig. 1, a plurality of the optical fibers and tubes, which are required to be used sequentially, can be preliminarily set in a waiting state at a location relatively adjacent to a diseased part in a thin vas. Thus, in case of necessity, only a necessary one of the optical fibers and tubes can be retractably inserted into the small diameter portion 2 selectively and immediately so as to be drawn close to the diseased part through common use of one duct.

Meanwhile, in the above embodiment, the catheter provided with the balloon for securing the catheter and for stopping blood flow has been described. Therefore, in the small diameter portion of the catheter, the duct as the gas passage for the balloon and the duct for the optical fiber for transmitting laser beams, etc. are juxtaposed. However, in case of diagnosis and therapy of a diseased part in a minute vas, this balloon becomes unnecessary. In this case, the duct as the gas passage for the balloon can be eliminated, so that only one duct for the optical fiber for transmitting laser beams, etc. may be formed in the small diameter portion of the catheter and thus, the catheter at the small diameter portion can be further reduced in diameter. The design of the diagnostic and therapeutic catheter of the present invention can be modified variously without departing from the scope of the present invention.

In accordance with the diagnostic and therapeutic catheter of the present invention, only a necessary one of the optical fibers and tubes can be retractably inserted from the large diameter portion into the small diameter portion selectively and immediately so as to be drawn close to the target diseased part through common use of at least one duct. Accordingly, the catheter of the present invention can achieve such a characteristic effect that diagnosis and therapy based on the ordinary operational procedures can be performed rapidly and positively even in case of a thin vas.

## Claims

1. A diagnostic and therapeutic catheter (1) accommodating a plurality of optical fibers (10, 11) and at least one tube (13) in its proximal end (3);
**characterized by**
the distal end extent (2) of the catheter being of reduced diameter compared with the diameter of said proximal end (3) of the catheter;
at least one duct (6) in said distal end extent (2);
said plurality of optical fibers (10, 11) and said at least one tube (13) in said proximal end confronting an inlet to said at least one duct (6) in said distal end extent (2) whereby any one of said optical fibers (10, 11) and said at least one tube (13) is selectively retractably insertable into said at least one duct (6) in said distal end extent (2).

2. A catheter as in Claim 1 and wherein the plurality of optical fibers comprise an optical fiber (10) for transmitting laser beams and an image optical fiber (11) for endoscopy.

3. A catheter as in Claim 1 or Claim 2 and wherein said proximal end (3) of the catheter contains a wire (12) selectively retractably insertable into said at least one duct (6) for controlling said distal end extent (2).

4. A catheter as in any one of Claims 1 to 3 comprising a balloon (7) fixed at the distal end of the catheter (1) and a gas duct (5) extending through said proximal end (3) and into said distal end extent (2) for supplying gas to said balloon (7).

5. A catheter as in any one of Claims 1 to 4 comprising a light guide (4) therein for illumination.

6. A catheter as in Claims 4 and 5 and wherein said light guide (4) occupies a gap between the peripheral surfaces of said gas duct (5) and said at least one duct (6) into which said plurality of optical fibers (10, 11); said at least one tube (13) are selectively retractably insertable.

## Patentansprüche

1. Diagnostischer und therapeutischer Katheter (1) mit einer Vielzahl von Lichtleitfasern (10, 11) und mindestens einer Röhre (13) in seinem proximalen Ende (3), dadurch gekennzeichnet, daß die distale Endausdehnung (2) des Katheters von geringerem Durchmesser verglichen mit dem Durchmesser von dem proximalen Ende (3) des Katheters ist; daß mindestens ein Kanal (6) in der distalen Endausdehnung (2) angeordnet ist; und daß die Vielzahl von Lichtleitfasern (10, 11) und die mindestens eine Röhre (13) in dem proximalen Ende einem Eingang zum mindestens einen Kanal (6) in der distalen Endausdehnung (2) , gegenüberliegend angeordnet sind, wobei jede der Lichtleitfasern (10, 11) und die mindestens eine Röhre (13) wahlweise ausziehbar, einschiebbar in dem mindestens einen Kanal (6) in der distalen Endausdehnung (2) sind.

2. Katheter nach Anspruch 1, dadurch gekennzeichnet, daß die Vielzahl der Lichtleitfasern eine Lichtleitfaser (10) zur Übertragung von Laserstrahlen und eine Bildlichtleitfaser (11) zur Endoskopie umfassen.

3. Katheter nach Anspruch 1 oder 2, dadurch gekennzeichnet, daß das proximale Ende (3) des Katheters einen wahlweise ausziehbaren, in dem mindestens einen Kanal (6) einschiebbaren Draht (12) zur Steuerung der distalen Endausdehnung (2) beinhaltet.

4. Katheter nach einem der Ansprüche 1 bis 3, gekennzeichnet durch einen Ballon (7), der am distalen Ende des Katheters (1) befestigt ist, und einen Gaskanal (5), der sich durch das proximale Ende (3) und in die distale Endausdehnung (2) zur Gasversorgung des Ballons (7) erstreckt.

5. Katheter nach einem der Ansprüche 1 bis 4, gekennzeichnet durch einen darin angeordneten Lichtleiter (4) zur Beleuchtung.

6. Katheter nach Anspruch 4 und 5, dadurch gekennzeichnet, daß der Lichtleiter (4) den Zwischenraum zwischen den Umfangflächen des Gaskanals (5) und des mindestens einen Kanals (6) füllt, in welchen Kanal die Vielzahl von Lichtleitfasern (10, 11) und die mindestens eine Röhre (13) wahlweise ausziehbar, einschiebbar sind.

## Revendications

1. Cathéter (1) à fins diagnostiques et thérapeutiques, dans lequel sont logés, dans son extrémité arrière (3), une pluralité de fibres optiques (10, 11), et au moins un tube (13);
caractérisé
par le fait que la zone d'extrémité avant (2) du cathéter est de diamètre réduit en comparaison du diamètre de ladite zone arrière (3) du cathéter;
par au moins un conduit (6) dans ladite zone d'extrémité avant (2);
par le fait que ladite pluralité de fibres optiques (10, 11) et ledit tube, dont il y a au moins un (13), font, dans ladite extrémité arrière, face à une entrée qui conduit à une entrée dudit conduit, dont il y a au moins un, (6) qui est dans ladite zone d'extrémité avant (2), ce par quoi l'une quelconque desdites fibres optiques (10, 11) et dudit tube (13) dont il y a au moins un peut s'insérer, sélectivement et de façon rétractable, dans ledit conduit, dont il y a au moins un, (6) qui est dans ladite zone d'extrémité avant (2).

2. Cathéter selon la revendication 1 et dans lequel la pluralité de fibres optiques comporte une fibre optique (10) pour transmettre des faisceaux laser et une fibre optique (11) pour image pour endoscopie.

3. Cathéter selon la revendication 1 ou la revendication 2 et dans lequel ladite extrémité arrière (3) du cathéter contient un conducteur (12) qui peut s'insérer, sélectivement et de façon rétractable, dans ledit conduit dont il y a au moins un (6) pour commander ladite zone d'extrémité avant (2).

4. Cathéter selon l'une quelconque des revendications 1 à 3, comportant un ballon (7) fixé à l'extrémité avant du cathéter (1) et un conduit de gaz (5) qui passe par ladite extrémité arrière (3) et vient dans ladite zone d'extrémité avant (2) pour alimenter en gaz ledit ballon (7).

5. Cathéter selon l'une quelconque des revendications 1 à 4, comportant en son intérieur, un guide de lumière (4)

6. Cathéter selon les revendications 4 et 5 et dans lequel ledit guide de lumière (4) occupe un jeu existant entre les surfaces périphériques dudit conduit de gaz (5) et dudit conduit (6), dont il y a au moins un, dans lesquels ladite pluralité de fibres optiques (10, 11) et ledit tube (13) dont il y a au moins un peuvent s'insérer de façon sélective et rétractable.
